# EUROPEAN PATENT APPLICATION

(11) **EP 3 777 685 A1**
(43) Date of publication of application: **17.02.2021**
(21) Application number: 20178142.4
(22) Date of filing: 27.11.2012
(51) Int. Cl.: A61B 5/20, A61F 2/00

(54) **TREATMENT OF URINARY INCONTINENCE**

(30) Priority: 28.11.2011 US 201161563889 P
(62) Divisional of application: 12852598.7
(71) Applicant: Remendium Labs LLC, LSU Box 25128, Baton Rouge, LA 70803 (US)
(72) Inventor: IGLESIAS, Ramon Jose, DeLeon Springs, Florida 32130 (US)
(74) Representative: EIP

(57) **Abstract**

The present invention relates to the diagnosis and treatment of urinary incontinence. The diagnosis and treatment involves the use of a multiple sensor-enabled catheter capable of providing real-time data regarding the patient's physiology, such as urinary flow and muscular function of the bladder of the bladder sphincter, as well as the position and movement of the catheter within the patient.

## Description

### BACKGROUND OF THE INVENTION

The present invention relates to the diagnosis and treatment of urinary incontinence. The diagnosis and treatment may involve the use of a multiple sensor-enabled catheter capable of providing real-time data regarding the patient's physiology, such as urinary flow and muscular function of the bladder sphincter, as well as the position and movement of the catheter within the patient.

Urinary incontinence (UI), also known as effort incontinence, may be due to multiple factors, including the insufficient strength of the pelvic floor muscles as well as the loss of, or damage to, the ligaments normally supporting the urethra and the bladder. The factors, alone or in combination, may lead to the most common etiology of UI, namely hypermobility of the bladder neck. UI may present the loss of small amounts of urine associated with coughing, laughing, sneezing, exercising or other movements that increase intra-abdominal pressure and thus increase pressure on the bladder. Intrinsic sphincteric deficiency is the inability of the bladder sphincter to contract sufficiently to keep the urine from flowing between the bladder and the urethra, which results in UI.

In women, physical changes resulting from pregnancy, childbirth, weight gain, and menopause often contribute to incontinence. UI can worsen during the week before the menstrual period. At that time, lowered estrogen levels may lead to lower muscular pressure around the urethra, increasing chances of leakage. The incidence of incontinence increases following menopause, similarly because of lowered estrogen levels. In high-level female athletes, effort incontinence occurs in all sports involving abrupt repeated increases in intra-abdominal pressure that may exceed perineal floor resistance.

It is thought that the principal cause of UI is pregnancy and childbirth and the consequent tearing of the tissues that support the bladder and urethra. In an attempt to correct this defect, various surgeries have been devised, all with the intent of repositioning the bladder and urethra to their proper place by either a vaginal or abdominal surgical approach. These surgical connections would be highly successful if the bladder and bladder neck could be restored to their natural position. Unfortunately, current surgeries have a high failure rate due to the lack of a definitive way of checking whether the positioning is correct during any of the surgeries.

Currently, the health care provider pulls the bladder neck, usually through the vaginal wall, into an approximated position to achieve the optimal, effective sphincteric pressure to prevent urine flow between the bladder and the urethra. The position is approximated because the health care provider is actually unable to determine the true, correct position to achieve this optimal, effective sphincteric pressure. The accuracy of this approximated position may be confirmed only through the passage of time and/or the willingness of the patient to complain about the UI or the recurrence of the UI, in which case, the patient would be subjected to yet another possibly unsuccessful surgery.

Each time a surgery is performed there is an increased amount of scar tissue. The general immobilization of the tissues will increase after subsequent surgeries, which will adversely affect the subsequent success/failure rate of these surgeries. There is a genuine need for the health care provider to be able to watch the urethra and the bladder and their positions in real-time as the surgery progresses in order to avoid more surgeries and to correct the UI during the initial procedure.

By knowing the patient's optimal anatomical positioning and sphineteric pressure in real-time, the health care provider would be able to position the urethra and the bladder neck correctly and not have to guess at the proper placement. By knowing the real-time pressure exerted by the bladder sphincter, the health care provider or patient can achieve the proper muscular function of the bladder sphincter, as well as correctly position the urethra and bladder neck, to relieve the UI. With this real-time capability, the diagnostic or therapeutic procedure would no longer be blind, which otherwise could, with little or no predictability, leave the urethra too tight or too loose or subject to the happenstance of a correct positioning.

One of the most accurate tools currently available for diagnosing UI is a cystourethrogram. The diagnoses of UI using this method are based on difficult to interpret pressure variants, which may lead to misdiagnoses of UI versus urge incontinence versus neurological defect. Often, presently available diagnostic methods test the patient in the dorso lithotomy position during which time UI does not occur.

A patient instead should be tested awake and in the office under the same circumstances that cause incontinence, such as coughing, running, jumping, etc., making the diagnosis of the etiology more accurate by monitoring the mobilization of the patient's urethra and bladder neck. This information may be relied upon in the operating room to enable the health care provider to manipulate the urethra and bladder neck vaginally into the optimal position.

### BRIEF SUMMARY OF THE INVENTION

The present invention relates to the diagnosis and treatment of UI. In an embodiment of the invention, this diagnosis and treatment involves the use of a multiple sensor-enabled catheter capable of providing real-time data regarding the patient's physiology, such as urinary flow and muscular function of the bladder sphincter, as well as the position and movement of the catheter within the patient. In one embodiment, the device may be a Foley catheter.

The multiple sensor-enabled catheter may include at least one sensor capable of providing real-time data of one or more types selected from the group consisting of position, movement, pressure, and flow. In this regard, a sensor may have a single measurement and reporting capability, or may have multiple measurement and reporting capabilities.

The present invention also includes a method for the diagnosis or treatment of UI comprising providing a multiple sensor-enabled catheter in a patient and determining the anatomical state of the patient capable of relieving the incontinence. The anatomical state may be the relative position of the bladder neck and urethra. The anatomical state may also be the muscular function of the bladder sphincter. The method of diagnosis or treatment may also include manipulating the patient to relieve the incontinence. The manipulation may be performed by the health care provider and/or the patient. The manipulation may include achieving a particular anatomical position of the bladder neck relative to the urethra and/or achieving a particular muscular function of the bladder sphincter.

The present invention contemplates the real-time position and movement tracking described in International Patent Application PCT/US2010/053712, which is hereby incorporated in its entirety by reference. In this regard, the real-time position and movement tracking may include sensing the position of the bladder relative to a fixed reference point within the patient by providing a catheter enabled with a sensor capable of providing positional and/or movement data. The fixed reference point within the body may be the pubic bone, the coccyx or the vagina. The method may be performed in real-time, for example, during an operation. In another embodiment, the method may be performed at multiple time intervals. The multiple time intervals may occur, for example, pre- and post-event, wherein the event may be pregnancy or menopause.

### BRIEF DESCRIPTION OF THE DRAWINGS

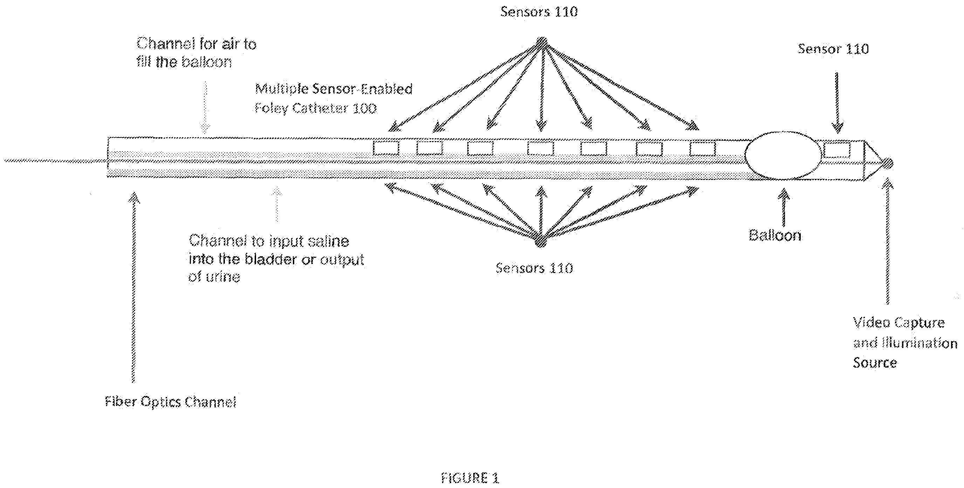

Figure 1 depicts a lateral view of an embodiment of the present invention.

### DETAILED DESCRIPTION OF THE INVENTION

When used in the claims, the terms "a" and "an" and "the" and similar references in the context of describing the invention (especially in the context of the following claims) are to be construed to cover both the singular and the plural, unless otherwise indicated herein or clearly contradicted by context. Also when used in the claims, the terms "comprising," "having," "including," and "containing" are to be construed as openended terms (i.e., meaning "including, but not limited to,") unless otherwise noted. To the extent used, the recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. Variations of the embodiments may become apparent to those of ordinary skill in the art upon reading the description. Accordingly, this invention includes all modifications and equivalents of the subject matter recited in the claims appended hereto as permitted by applicable law. Moreover, any combination of the described elements in all possible variations thereof is encompassed by the invention unless otherwise indicated herein or otherwise clearly contradicted by context.

For purposes of the present invention, the term "urethra" may be defined as the canal leading from the bladder, discharging the urine externally. See STEDMAN'S MEDICAL DICTIONARY, at page 2072 (28th ed.). In females, the urethra is a canal about 4 centimeters long passing from the bladder, in close relation with the anterior wall of the vagina and having a long axis that parallels that of the vagina opening in the vestibule of the vagina posterior to the clitoris and anterior to the vaginal orifice. *Id.* The term "urinary bladder" refers to a musculomembranous elastic bag serving as a storage place for the urine, filled via the ureters and drained via the urethra. *Id*. at page 226. The term "bladder neck" is defined as the smooth muscle of the bladder neck is histologically, histochemically and pharmacologically distinct from the detrusor muscle proper and so the bladder neck should be considered as a separate functional unit. *See* GRAY'S ANATOMY, at page 1290 (39th ed.). The arrangement of smooth muscle in this region is quite different in males and females, and therefore each sex is described separately. In females, the bladder neck consists of morphologically distinct smooth muscle. The large diameter fasciculi characteristic of the detrusor is replaced in the region of the bladder neck by small diameter fasciculi which extend obliquely or longitudinally into the urethral wall. *Id.* In the normal female the bladder neck which above the pelvic floor supported predominantly by the pubovesical ligaments, the endopelvic fascia of the pelvic floor and levator ani. These support the urethra at rest; with elevated intra-abdominal pressure the levators contract increasing urethral closure pressure to maintain continence. This anatomical arrangement commonly alters after parturition and with increasing age, such that the bladder neck lies beneath the pelvic floor, particularly when the intra-abdominal pressure rises. The mechanism described above may fail to maintain continence (incontinence as a result of urethral hypermobility).

In the present invention, for example, a Foley catheter could be enabled with at least one sensor capable of providing real-time data of one or more types selected from the group consisting of position, movement, pressure, and flow. In this regard, a sensor may have a single measurement and reporting capability, or may have multiple measurement and reporting capabilities. The data obtained by the multiple sensor-enabled catheter may be reported in any number of ways know in the art, including the transmission to, and visualization on, a graphical user interface. Below the firm tip of the Foley catheter, e.g., about ½ inch, a small section of the device would be filled with normal saline solution in order to find the neck of the bladder. For purposes of the invention, "real-time" may include instantaneous as well as delayed observation, reporting and/or recording of an event as it elapses.

By operating with an image of where the bladder and urethra are in the patient relative to the pubic bone, the coccyx or the vagina in real-time during the procedure, the health care provider would be able to pull the bladder and the urethra to a position considered normal under direct observation and not merely by guessing how tight or how loose to position the anatomy.

The multiple sensor-enabled catheter would be invaluable as a study or diagnostic tool for the health care provider as well as the patient who is considering a pregnancy. The health care provider may be able to provide the patient with an in-office procedure that would determine a baseline position and a relative mobilization of the bladder (baseline) before the possible damage to her pelvic floor that may occur during pregnancy and delivery, so when the surgical repair, if needed, is performed, her bladder can be repositioned to the original, pre-incontinence anatomic position. Surgery could be performed on patients with a surgically correctable structural defect.

The multiple sensor-enabled catheter would also help with any diagnosis where surgery is an option and the position of the bladder needs to be adjusted surgically to correct any urinary problem, such as that involving a woman who cannot empty her bladder due to avulsion of the bladder through the vagina. In males, prostactic hypertrophy causes a stricture of the urethra. The diagnosis of urethral stricture is usually made by the patient's history and confirmed after an operative cystogram. The diagnosis of urethral stricture could be made by using a multiple sensor-enabled catheter much like an ovarian cyst is confirmed by a sonogram and not by an operative laparoscopy. The multiple sensor-enabled catheter may also be used in a male after a prostatectomy. In this context, as with a female patient, the multiple sensor-enabled catheter may be used to help determine the optimal positioning of the urethra and bladder neck and the pressure exerted by the bladder sphincter.

Another use for a multiple sensor-enabled catheter would be to correct fecal incontinence, which is often another sequela of pregnancy and childbirth. An elongated rather than round apparatus would be inserted into the rectum and a different, but similar apparatus would be inserted into the vagina and the multiple sensor-enabled catheter would be inserted into the bladder and all related to the pubic bone for correct positioning. With this information the health care provider would be able to properly position the anatomy surgically, in real-time, therefore correcting the fecal incontinence.

A proper diagnosis of a surgically treatable case of UI may be obtained by using the multiple sensor-enabled catheter. In a vaginal approach surgery, the health care provider would insert the multiple sensor-enabled catheter in the bladder and allow it to drain. The anterior vaginal wall would be peeled off exposing the urethra and the bladder neck. Sutures or a sling would be placed and held until ready. The Foley balloon would be inflated and its position inside the bladder would be measured relative to the pubic bone, coccyx or vagina, and the predetermined, optimal position recorded in real-time. The health care provider would then tighten the sutures visualizing the previously obtained optimal position of the urethra and bladder neck. The health care provider would then tighten the sutures, visualizing the elevation of the bladder and the bladder neck on the computer screen until the correct position is achieved. In the past, this position has only been approximated by the health care provider, which may explain the high failure rate of this procedure.

UI is thought to occur following the damage to the ligaments holding the urethra and bladder neck to their anatomical positions. As these anatomical changes occur, it is unknown if the positional changes affect the contracting ability of the bladder sphincter muscles. By virtue of simple physical principles, it is known that there is a direct relationship between degree of stretching an elastic tube (the urethra in this case) and the circumferential pressure imparted to the annulus (composed of the bladder sphincter muscles). By incorporating pressure sensors in the catheter along with positional sensors, the intrinsic force on the bladder sphincter could be measured as a consequence of the positional differences of the urethra and bladder neck.

By placing pressure sensors along the walls of the catheter, one could measure the intrinsic pressure of the bladder sphincter as well as the pressure imparted on the midurethral "muscular bundle" in males and females if this "muscular bundle" exists and has any effect in establishing continence.

By combining flow sensors along the catheter along with the positional sensors and the pressure sensors, objective measurements relating urine flow, sphincter pressure and positional location can be evaluated and correlated to aid in the diagnosis and treatment of UI.

By placing a visual sensor (e.g., a camera, a fiber optic lens or other such) device in the catheter, the real-time events can be recorded as they occur. This could establish an objective "map" to aid the health care provider to diagnose the cause of incontinence and suggest the best treatment modalities for the patient's incontinence.

UI has two different etiologies. One is hypermobility of the urethra and the other is intrinsic sphincter deficiency. One method of treating UI surgically is by attaching a sling or modifying the positioning of the urethra. UI can be surgically treated by attaching a sling, modifying the urethra position and/or changing the circumferential pressure induced upon the bladder sphincter by tensioning the urethra. From these factors, it is deduced that positioning influences UI. However, it is not known what changes are induced by repositioning the urethra or the bladder neck and what or how these factors directly affect UI. By introducing pressure sensors within the urethra using a multiple sensor-enabled catheter; monitoring the intra-urethral pressures imparted by bladder sphincter; and tracking the positioning/repositioning of the urethra and the bladder neck in real-time, the multiple sensor-enabled catheter may assist health care providers in relating anatomical positions to controlling flow of urine. The proper anatomical positioning may have a direct effect on the ability of the sphincter and perimuscular bands surrounding the urethra to contract more effectively thereby controlling continence/incontinence. While presently available urodynamics assessments may allow the recording of differential pressures between the bladder and urethra, these assessments fail to offer any information on the anatomical positions affecting continence or incontinence.

The multiple sensor-enabled catheter may incorporate at least one sensor capable of measuring and/or reporting data of various types including position, movement, pressure and flow. A multiple sensor-enabled catheter with more than one individual sensor may be arrayed as depicted in Figure 1. However, a multiple sensor-enabled catheter may incorporate a single sensor capable of multiple measurement and reporting capabilities.

The position and movement data may be of the sort measured and/or reported by any number of sensor devices, including an accelerometer, gyroscope, inductive noncontact position sensor, string potentiometer, linear variable differential transformer, potentiometer, capacitive transducer, Eddy-current sensor, Hall effect sensor, optical proximity sensor, piezo-electric transducer and photodiode array. The position and movement data may also include magnetic, electromagnetic, microelectromechanical, radio frequency, ultrasound and video.

The pressure and flow data may be of the sort measured and/or reported by any number of sensor devices, including force collector types, such as piezo-resistive, capacitive, electromagnetic, piezo-electric, optical, potentiometric, or other types, such as resonant, thermal, ionization, ultrasonic, and density (mass and index of refraction).

Figure 1 depicts a Foley catheter (100) with a firm tip, which may be about ½ inch in length to guide the Foley catheter through the urethra. There is a small hole in this tip portion to facilitate drainage of any urine in the patient's bladder. Proximal from the tip is a section that comprises an inflatable balloon, which may be at least 10 cc in volume (e.g., about 100 cc). The number and precise placement of an individual sensor (110) may vary depending on the type of positional, movement, pressure or flow measurement and/or reporting system employed. An individual sensor (110) may have a single function or be multifunction (such as positional tracking combined with pressure and flow sensing). The multiple sensor-enabled Foley catheter may also embody a video observation and/or recording device as well as an illumination source to facilitate such video capture. The precise placement of the sensor(s) and video capture component(s) are not pre-defined, and may be configured according to the requirements of the desired application.

### SPECIFIC EXAMPLES

As described earlier, the catheters of the present invention may embody at least one sensor capable of measuring and reporting at least one data type, including position, movement, pressure, and flow. These include, but are not limited to, magnetic, electromagnetic, microelectromechanical, radio frequency, ultrasound and video. One example of a multiple sensor-enabled catheter is a Foley catheter containing various microelectromechanical (MEMS) devices: a 3-axis accelerometer, a roll/pitch gyroscope and a yaw rate gyroscope, and a pressure and flow transducer. The devices may be mounted on a small flexible printed circuit board (PCB) and then attached to the Foley catheter. The 3-axis accelerometer tracks translation of the Foley catheter in three directions. The gyroscopes are utilized to account for gravitational rotation, allowing real-time movement to be tracked.

A PCB is prepared with the three MEMS devices mounted thereon. Soft leads trail the MEMS devices to supporting devices, including, for example, a data acquisition card which may be used for transforming analog signals to digital signals. The PCB is set within the wall of the Foley catheter. The location of the Foley catheter may be determined by the output signals of the MEMS devices.

The catheter may be inserted through the length of the urethra into bladder at which point the Foley catheter's balloon is inflated and the catheter is pulled in a proximal direction so that the balloon is located exactly above the bladder sphincter.

The patient may be asked to recreate maneuvers that induce incontinence at the same time that the parameters for the location/pressure/flow/visualization of the urethra and bladder are determined.

The urethra and bladder are manipulated to the position where muscular pressure is optimized and urine flow is returned to normal physiological control. These positions for the urethra and bladder neck may be displayed in real-time on a graphical user interface and/or recorded.

At the time of surgery, the urethra and bladder neck are repositioned to the location where the patient was previously determined to be continent (if pre-incontinence location was determined). If no pre-incontinence position is known, the best location of these anatomies will be established by utilizing data taken from a cohort of patients with similar UI history and profile.

Following the examination using the multiple sensor-enabled catheter, the health care provider may conclude that rehabilitation is an efficacious option for the patient. In this regard, the measurements provided by the multiple sensor-enabled catheter may be recorded to facilitate appropriate patient instructions on performing Kegel exercises in an optimal manner using the visual (on-screen) information provided by the catheter in real-time. Once engaging the proper musculature has been successfully communicated to the patient during the medical office visit, the patient may be sent home with the instructions to perform Kegel exercises five to six times daily, for example. Four to six weeks later the patient may return for another examination using the multiple sensor-enabled catheter to evaluate rehabilitative treatment effectiveness, which may allow the health care provider to advise the patient about the prospects for restoring complete continence with a continued rehabilitation regime and/or a surgical procedure.

Detailed embodiments of the present invention are disclosed herein; however, it is to be understood that the disclosed embodiments are merely exemplary of the invention that may be embodied in various forms. It will be appreciated that many modifications and other variations that will be appreciated by those skilled in the art are within the intended scope of this invention as claimed below without departing from the teachings, spirit and intended scope of the invention.

The invention will now be described by reference to the following numbered embodiments:
1. A device comprising a catheter having at least one sensor capable of providing real-time data of one or more types selected from the group consisting of position, movement, pressure, and flow.
2. The device of embodiment 1, wherein said catheter is a Foley catheter.
3. A method for the diagnosis or treatment of UI comprising providing the device of embodiment 1 in a patient and determining the anatomical state of the patient capable of relieving the incontinence.
4. The method of embodiment 3, wherein the anatomical state is the relative position of the bladder neck and urethra.
5. The method of embodiment 3, wherein the anatomical state is the muscular function of the bladder sphincter.
6. The method of embodiment 3 further comprising manipulating the patient to relieve the incontinence.

## Claims

1. A catheter comprising a plurality of microelectromechanical sensors positioned along a length of the catheter and capable of providing real-time position and/or movement data for sensing the position of the bladder relative to a fixed reference point within a subject, wherein the catheter comprises a plurality of pressure sensors positioned along a wall of the catheter and capable of providing real-time pressure data, wherein the real-time pressure data comprises pressure imparted by the bladder sphincter.

2. The catheter of claim 1, wherein the fixed reference point is the pubic bone, the coccyx or the vagina.

3. The catheter of claim 1 or 2, wherein the catheter is a Foley catheter.

4. The catheter of any one of claims 1-3, wherein the catheter further comprises at least one flow sensor capable of providing real-time flow data.

5. The catheter of claim 4, wherein the catheter comprises a plurality of flow sensors, positional sensors, and pressure sensors.

6. The catheter of any one of claims 1-5, further comprising a visual sensor.

7. The catheter of claim 6, wherein the visual sensor is a camera or a fiber optic lens.

8. The catheter of any one of claims 1-7, further comprising a balloon.

9. Use of the catheter of any one of claims 1-8 to obtain real-time data selected from position, movement, pressure, and flow.

10. The use of claim 9 wherein the real-time pressure data is related to the pressure of the bladder sphincter.
